Europäisches Patentamt

**(19)** European Patent Office

Office européen des brevets

**(11)** Publication number : **0 404 643 B1**

**(12)** # EUROPEAN PATENT SPECIFICATION

**(45)** Date of publication of patent specification :
**17.03.93 Bulletin 93/11**

**(51)** Int. Cl.⁵ : **C08F 6/28,** C08F 6/16

**(21)** Application number : **90401660.7**

**(22)** Date of filing : **14.06.90**

**(54) Method for removing mercaptans.**

**(30)** Priority : **22.06.89 JP 158384/89**
**05.07.89 JP 171898/89**

**(43)** Date of publication of application :
**27.12.90 Bulletin 90/52**

**(45)** Publication of the grant of the patent :
**17.03.93 Bulletin 93/11**

**(84)** Designated Contracting States :
**BE DE FR GB NL**

**(56)** References cited :
**DE-A- 2 144 567**
**GB-A- 2 196 982**
**US-A- 2 988 499**

**(73)** Proprietor : **MITSUBISHI RAYON CO., LTD**
**3-19, Kyobashi 2-chome Chuo-ku**
**Tokyo (JP)**

**(72)** Inventor : **Yoshioka, Teruhiko, c/o Mitsubishi**
**Rayon Co. Ltd.**
**20-1, Miyukicho**
**Otake-shi, Hiroshima 739-06 (JP)**
Inventor : **Okamura, Kouhei, c/o Mitsubishi**
**Rayon Co. Ltd.**
**20-1, Miyukicho**
**Otake-shi, Hiroshima 739-06 (JP)**
Inventor : **Kobayashi, Masao, c/o Mitsubishi**
**Rayon Co. Ltd.**
**20-1, Miyukicho**
**Otake-shi, Hiroshima 739-06 (JP)**

**(74)** Representative : **Bourgognon, Jean-Marie et al**
**Cabinet Flechner 22, Avenue de Friedland**
**F-75008 Paris (FR)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 404 643 B1

## Description

The present invention relates to a method for removing mercaptans from acrylates or methacrylates containing a small amount of mercaptans as an impurity.

When, for example, acrylates or methacrylates are polymerized using peroxides or azo compounds as polymerization initiators and mercaptan compounds as chain transfer agents and then the remaining monomers are recovered by distillation, the recovered monomers are usually difficult to be reused as they are, since they contain mercaptans which are left unremoved. The presence of mercaptans, even if an amount thereof is very small, is so smelly that it causes problems in handling. Washing with sulfuric acid, alkalis or water is usual for removal of mercaptans contained in hydrocarbons, aromatic compounds and the like. However, these methods require complicated steps and besides have the limitation that only such liquid as inert to sulfuric acid or alkali can be used. It is also difficult to completely and selectively remove only mercaptans by extraction or distillation.

Therefore, development of a simple and efficient method for removing mercaptans contained as an impurity has been demanded.

The present invention is a method for removing mercaptans, characterized by treating acrylates or methacrylates containing mercaptans as an impurity with at least one compound selected from the group consisting of lead compounds and copper compounds.

As the acrylates or methacrylates, mention may be made of, for example, methyl acrylate, ethyl acrylate, propyl acrylate, butyl acrylate, methyl methacrylate, ethyl methacrylate, propyl methacrylate, butyl methacrylate and mixtures thereof.

As the mercaptans contained in acrylates or methacrylates, mention may be made of, for example, alkyl mercaptans of 1-8 carbon atoms such as methyl mercaptan, ethyl mercaptan, propyl mercaptan, butyl mercaptan, and octyl mercaptan and mixtures thereof. These are mainly the compounds added as chain transfer agents in polymerization of acrylates or methacrylates.

Content of the mercaptans which are impurities is usually about 0.3% by weight or less, but may be more than 0.3% by weight.

The method of the present invention will be explained with reference to removal of mercaptans from acrylates or methacrylates containing a small amount of mercaptans as an impurity.

The lead compounds used in the present invention include, for example, trilead tetraoxide, lead monoxide, lead dioxide and mixtures thereof. Preferred is trilead tetraoxide.

The copper compounds include, for example, copper (II) carboxylates, especially preferably copper (II) carboxylates of 2-19 carbon atoms such as copper formate, copper acetate, copper citrate, copper oleate and copper naphthenate. Further examples are inorganic copper salts such as copper sulfate and copper chloride, complex compounds such as copper (II) acetylacetonate and mixtures thereof. These lead compounds and copper compounds may be used alone or in combination to exhibit the desired effect. These are used preferably in the form of powder or granules.

The lead compounds and/or copper compounds are preferably used in an amount of 0.05-5 parts by weight in terms of lead and/or copper per part by weight of mercaptans contained as an impurity. Treating temperature is 0-60°C, preferably from room temperature to 40°C and treating time is preferably 1 to several hours.

Under these conditions, the materials containing mercaptans and the lead compound and/or copper compound are kept for a given time preferably under stirring.

By this treatment, mercaptans contained as an impurity can be substantially removed without any adverse effect on acrylates or mehacrylates.

The resulting treated liquid can be further purified by distillation. In this case, the distillation can be conducted after the lead compounds and/or copper compounds are removed by filtration or centrifugal separation. Alternatively, the lead compounds and/or copper compounds may not be removed before the distillation.

In this distillation, polymerization inhibitors generally used for inhibition of polymerization of acrylates or methacrylates can be preferably used.

The present invention will be explained in more detail by the following examples.

Example 1

In a 300 ml three-necked flask equipped with a stirrer and a cooler was charged 200 g of methyl methacrylate containing 1400 ppm of n-butyl mercaptan and was added thereto 0.6 g of trilead tetraoxide. This flask was dipped in a constant temperature bath of 25°C and stirred at 300-400 $min^{-1}$.

After 2 hours, 500 ppm of diphenyl-p-phenylene-diamine was added as a polymerization inhibitor. A Liebig condenser was provided and temperature of the constant temperature bath was elevated to 60°C and methyl methacrylate was distilled under reduced pressure (180 mmHg ≙ 24 kPa). Distillate obtained after distillation of about 98% was subjected to determination of amount of n-butyl mercaptan by flame ionization detector type gas chromatography to find that the amount was less than the minimum amount for assay (10 ppm).

Example 2

Example 1 was repeated except that 0.8 g of copper naphthenate was used in place of the trilead tetraoxide. n-Butyl mercaptan was not detected in the distillate.

Example 3

Example 1 was repeated except that an amount of the trilead tetraoxide was changed from 0.6 g to 0.2 g and 0.4 g of copper naphthenate was further added. n-Butyl mercaptan was not detected in the distillate.

Examples 4-11

Example 1 was repeated except that the lead compound or the copper compound as shown in Table 1 was used and the acrylate or methacrylate as shown in Table 1 was added. Amounts of mercaptan in the resulting acrylate or methacrylate are also shown in Table 1.

Table 1

| Example- | Acrylate or meth- acrylate | Mercaptan (ppm) | Lead Compound or copper compound | Mercaptan in distillate after treatment (ppm) |
|---|---|---|---|---|
| 4 | Methyl meth- acrylate | n-Butyl mercaptan (1400) | Copper acetate 0.5 g | less than 10 |
| 5 | ditto | ditto | Copper (II) acetyl- acetonate 0.6 g | ditto |
| 6 | ditto | ditto | Copper citrate 0.8 g | ditto |
| 7 | Methyl acrylate | n-Butyl mercaptan (1000) | Trilead tetraoxide 0.5 g | ditto |
| 8 | ditto | ditto | Copper naphthenate 0.7 g | ditto |
| 9 | Butyl meth- acrylate | ditto | Trilead tetraoxide 0.5 g | ditto |
| 10 | Methyl meth- acrylate | Methyl mercaptan (1000) | Copper naphthenate 0.7 g | ditto |
| 11 | ditto | ditto | Trilead tetraoxide 0.5 g | ditto |

## Claims

1. A method for removing mercaptan which comprises treating acrylates or methacrylates containing mercaptans as an impurity with at least one compound selected from the group consisting of lead compounds and copper compounds.

2. A method according to claim 1, wherein the acrylates or methacrylates are methyl acrylate, ethyl acrylate, propyl acrylate, butyl acrylate, methyl methacrylate, ethyl methacrylate, propyl methacrylate, butyl methacrylate or mixtures thereof.

3. A method according to claim 1 or 2, wherein the mercaptans are alkyl mercaptans of 1-8 carbon atoms.

4. A method according to claim 3, wherein the mercaptans are methyl mercaptan, ethyl mercaptan, propyl mercaptan, butyl mercaptan, octyl mercaptan or a mixture thereof.

5. A method according to claims 1 to 4, wherein the content of the mercaptans is 0.3 % by weight or less.

6. A method according to claims 1 to 5, wherein the lead compounds are trilead tetraoxide, lead monoxide, lead dioxide or a mixture thereof.

7. A method according to claims 1 to 6, wherein the copper compounds are copper (II) carboxylates, inorganic copper salts, complex compounds of copper or a mixture thereof.

8. A method according to claim 7, wherein the copper compounds are copper (II) carboxylates of 2-19 carbon atoms.

9. A method according to claim 8, wherein the copper (II) carboxylates of 2-19 carbon atoms are copper formate, copper acetate, copper citrate, copper oleate, copper naphthenate or a mixture thereof.

10. A method according to claim 7, wherein the inorganic copper salts are copper sulfate, copper chloride or a mixture thereof.

11. A method according to claim 7, wherein the complex compounds of copper are copper (II) acetylacetonate.

12. A method according to claims 1 to 11, wherein the amount of at least one compound selected from the group consisting of lead compounds and copper compounds is 0.05-5 parts by weight in terms of lead and copper per part by weight of mercaptans.

13. A method according to claims 1 to 12, wherein the treating is effected at 0-60°C.

14. A method according to claim 13, wherein the treating is effected at a temperature from room temperature to 40°C.

15. A method according to claims 1 to 14, wherein the treating is effected for one hour or more.

16. A method according to claims 1 to 15, wherein liquid after the treatment is subjected to distillation after or before the lead compounds and copper compounds are removed.

## Patentansprüche

1. Verfahren zur Entfernung von Mercaptan, umfassend die Behandlung von Acrylaten oder Methacrylaten, welche Mercaptane als Verunreinigung enthalten, mit mindestens einer Verbindung, ausgewählt aus der Gruppe, bestehend aus Bleiverbindungen und Kupferverbindungen.

2. Verfahren gemäss Anspruch 1, worin die Acrylate oder Methacrylate Methylacrylat, Ethylacrylat, Propylacrylat, Butylacrylat, Methylmethacrylat, Ethylmethacrylat, Propylmethacrylat, Butylmethacrylat oder deren Mischungen sind.

3. Verfahren gemäss Anspruch 1 oder 2, worin die Mercaptane Alkylmercaptane mit 1 bis 8 Kohlenstoffatomen sind.

4. Verfahren gemäss Anspruch 3, worin die Mercaptane Methylmercaptan, Ethylmercaptan, Propylmercaptan, Butylmercaptan, Octylmercaptan oder eine Mischung davon sind.

5. Verfahren gemäss den Ansprüchen 1 bis 4, worin der Gehalt der Mercaptane 0,3 Gew.% oder weniger beträgt.

6. Verfahren gemäss den Ansprüchen 1 bis 5, worin die Bleiverbindungen Tribleitetroxid, Bleimonoxid, Bleidioxid oder eine Mischung davon sind.

7. Verfahren gemäss den Ansprüchen 1 bis 6, worin die Kupferverbindungen Kupfer(II)carboxylate, anorganische Kupfersalze, Komplexverbindungen von Kupfer oder eine Mischung davon sind.

8. Verfahren gemäss Anspruch 7, worin die Kupferverbindungen Kupfer(II)carboxylate mit 2 bis 19 Kohlenstoffatomen sind.

9. Verfahren gemäss Anspruch 8, worin die Kupfer(II)carboxylate mit 2 bis 19 Kohlenstoffatomen Kupferformiat, Kupferacetat, Kupfercitrat, Kupferoleat, Kupfernaphthenat oder eine Mischung davon sind.

10. Verfahren gemäss Anspruch 7, worin die anorganischen Kupfersalze Kupfersulfat, Kupferchlorid oder eine Mischung davon sind.

11. Verfahren gemäss Anspruch 7, worin die Kupferkomplexverbindung Kupfer(II)acetylacetonat ist.

12. Verfahren gemäss den Ansprüchen 1 bis 11, worin die Menge mindestens einer Verbindung, ausgewählt aus der Gruppe, bestehend aus Bleiverbindungen und Kupferverbindungen, 0,05 bis 5 Gew.-Teile, bezogen auf Blei und Kupfer, pro Gew.-Teil Mercaptan beträgt.

13. Verfahren gemäss den Ansprüchen 1 bis 12, worin die Behandlung bei 0 bis 60°C ausgeführt wird.

14. Verfahren gemäss Anspruch 13, worin die Behandlung bei einer Temperatur von Raumtemperatur bis 40°C ausgeführt wird.

15. Verfahren gemäss den Ansprüchen 1 bis 14, worin die Behandlung 1 Stunde oder länger durchgeführt wird.

16. Verfahren gemäss den Ansprüchen 1 bis 15, worin die Flüssigkeit nach der Behandlung destilliert wird, nachdem oder bevor die Bleiverbindungen und Kupferverbindungen entfernt worden sind.


**Revendications**

1. Procédé d'élimination d'un thiol, qui consiste à traiter des acrylates ou des méthacrylates contenant des thiols comme impureté, par au moins un composé choisi dans le groupe consistant en les composés de plomb et les composés de cuivre.

2. Procédé suivant la revendication 1, dans lequel les acrylates ou les méthacrylates sont l'acrylate de méthyle, l'acrylate d'éthyle, l'acrylate de propyle, l'acrylate de butyle, le méthacrylate de méthyle, le méthacrylate d'éthyle, le méthacrylate de propyle, le méthacrylate de butyle ou leurs mélanges.

3. Procédé suivant la revendication 1 ou 2, dans lequel les thiols sont des alcoylthiols ayant de 1 à 8 atomes de carbone.

4. Procédé suivant la revendication 3, dans lequel les thiols sont le méthylthiol, l'éthylthiol, le propylthiol, le butylthiol, l'octylthiol ou leurs mélanges.

5. Procédé suivant la revendication 1 à 4, dans lequel la teneur en thiols est de 0,3 % en poids ou est inférieure à 0,3 % en poids.

6. Procédé suivant l'une des revendications 1 à 5, dans lequel les composés de plomb sont le minium, le monoxyde de plomb, le dioxyde de plomb ou leurs mélanges.

7. Procédé suivant l'une des revendications 1 à 6, dans lequel les composés de cuivre sont des carboxylates de cuivre (II), des sels de cuivre minéraux, des complexes de cuivre ou leurs mélanges.

8. Procédé suivant la revendication 7, dans lequel les composés de cuivre sont des carboxylates de cuivre (II) ayant de 2 à 19 atomes de carbone.

9. Procédé suivant la revendication 8, dans lequel les carboxylates de cuivre (II) ayant de 2 à 19 atomes de carbone sont le formiate de cuivre, l'acétate de cuivre, le citrate de cuivre, l'oléate de cuivre, le naphténate de cuivre ou leurs mélanges.

10. Procédé suivant la revendication 7, dans lequel les sels de cuivre minéraux sont le sulfate de cuivre, le chlorure de cuivre ou leurs mélanges.

11. Procédé suivant la revendication 7, dans lequel les composés complexes de cuivre sont l'acétylacétonate de cuivre (II).

12. Procédé suivant l'une des revendications 1 à 11, dans lequel la quantité d'au moins un composé choisi parmi le groupe consistant en des composés de plomb et des composés de cuivre est de 0,05 à 5 parties en poids exprimées en plomb et en cuivre par partie en poids de thiols.

13. Procédé suivant l'une des revendications 1 à 12 qui consiste à effectuer le traitement entre 0 et 60°C.

14. Procédé suivant l'une des revendications 13, qui consiste à effectuer le traitement à une température allant de la température ambiante à 40°C.

15. Procédé suivant l'une des revendications 1 à 14, qui consiste à effectuer le traitement pendant une heure ou pendant plus d'une heure.

16. Procédé suivant l'une des revendications 1 à 15, qui consiste à soumettre le liquide après le traitement à une distillation avant ou après que les composés de plomb et que les composés de cuivre ont été éliminés.